# EUROPEAN PATENT APPLICATION

(11) **EP 3 932 329 A1**
(43) Date of publication of application: **05.01.2022**
(21) Application number: 21183071.6
(22) Date of filing: 01.07.2021
(51) Int. Cl.: A61B 17/072, A61B 8/08

(54) **SURGICAL STAPLING DEVICE**

(30) Priority: 02.07.2020 US 202016919168
(71) Applicant: Covidien LP, Mansfield, MA 02048 (US)
(72) Inventor: DIAZ-CHIOSA, Olesea, Naugatuck, CT 06770 (US)
(74) Representative: Maschio, Antonio

(57) **Abstract**

Surgical stapling devices have a first buttress attached to an anvil jaw member. The first buttress has a biocompatible contrast agent which elutes into tissue that is stapled, permitting intraoperative identification of cancerous cells in tissue remaining in a patient. In aspects, the surgical stapling device has a second buttress attached to a cartridge jaw member, which may also have a biocompatible contrast agent.

## Description

### TECHNICAL FIELD

The present disclosure relates to medical devices, including surgical devices such as buttresses, for use with wound closure devices. Medical devices formed of the materials of the present disclosure are capable of delivering diagnostic agents to a patient.

### BACKGROUND

Surgical stapling instruments are employed by surgeons to sequentially or simultaneously apply one or more rows of fasteners, e.g., staples or two-part fasteners, to body tissue for the purpose of joining segments of body tissue together. Such instruments generally include a pair of jaws or finger-like structures between which the body tissue to be joined is placed. When the stapling instrument is actuated, or "fired", longitudinally moving firing bars contact staple drive members in one of the jaws. The staple drive members push the surgical staples through the body tissue and into an anvil in the opposite jaw, which forms the staples. If tissue is to be removed or separated, a knife blade can be provided in the jaws of the device to cut the tissue between the lines of staples.

For some surgical procedures, it may be desirable to introduce diagnostic agents at the site of treatment. For example, in surgical procedures to remove cancerous tissues, a surgeon will remove the cancerous tissue and the patient will be periodically monitored for cancer recurrence. The extent of surgical resection of cancerous tissue is determined by biopsy results. If the margin of resected tissue is positive for cancer cells, the tissue is further resected, and a greater portion of tissue is removed.

Biopsy analysis is limited to a resected sliver of tissue; there is no insight on the state of the tissue remaining inside the patient. There is a possibility that cancer cells have migrated beyond resection sites but have not reached the lymph nodes or other portions of the patient's body. These cells have the potential to become new tumor-growing sites and normally will not be detected in traditional surgery.

Improved surgical repair materials, capable of use as buttresses for sealing and/or reinforcing staple lines against tissue, and improved methods for diagnosing conditions in a patient, including the presence of cancerous cells/tissues, remain desirable.

### SUMMARY

The present disclosure relates to medical devices, including surgical stapling devices, which can be used to repair tissue.

In aspects, the disclosed surgical stapling device includes an end effector having an anvil jaw member and a staple cartridge jaw member pivotally coupled to one another, the anvil jaw member and the staple cartridge jaw member being relatively movable such that the end effector is movable between an open position and a clamped position. A first buttress is attached to the anvil jaw member, the first buttress including at least one biocompatible contrast agent.

In some aspects, a second buttress is attached to the cartridge jaw member. The second buttress does not have to possess a biocompatible contrast agent. In aspects, the second buttress has at least one biocompatible contrast agent.

In aspects, the at least one biocompatible contrast agent is an artificial oxygen carrier selected from hemoglobin-based oxygen carriers, perfluorocarbon-based oxygen carriers, or combinations thereof.

In some aspects, the biocompatible contrast agent is combined with an excipient including a surfactant, a salt, an acid, a stabilizer, a polyhydric alcohol, a hydrotrope, a low molecular weight poly(ethylene glycol) or any combination thereof.

In aspects, the surfactant is a cyclodextrin, sodium dodecyl sulfate, octyl glucoside, a sorbitan fatty acid ester, or combinations thereof.

The salt can include sodium chloride.

In some aspects, the acid includes oleic acid, citric acid, ascorbic acid, or combinations thereof.

In other aspects, the stabilizer includes butylated hydroxytoluene, or butylated hydroxyanisole.

In aspects, the polyhydric alcohol includes D-sorbitol, mannitol, or combinations thereof.

In some aspects, the first buttress is attached to the anvil jaw member by at least one suture.

In other aspects, the second buttress is attached to the staple cartridge jaw member by at least one suture.

In aspects, the first buttress further includes a therapeutic agent selected from amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs, vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, anti-inflammatory agents, anti-bacterial agents, antimicrobial agents, and ribozymes.

In other aspects, a surgical stapling device of the disclosure includes an end effector including an anvil jaw member and a staple cartridge jaw member pivotally coupled to one another, the anvil jaw member and the staple cartridge jaw member being relatively movable such that the end effector is movable between an open position and a clamped position. A first buttress is attached to the anvil jaw member, the first buttress having at least one biocompatible contrast agent, and a second buttress is attached to the cartridge jaw member, the second buttress having at least one biocompatible contrast agent.

Methods for stapling tissue with the stapling device of the present disclosure are also provided.

### BRIEF DESCRIPTION OF THE DRAWINGS

Aspects of the presently disclosed surgical stapling apparatus are described herein with reference to the drawings wherein:
FIG. 1 is a perspective view of a surgical stapling apparatus including a handle housing, an adapter assembly, an end effector, and buttresses attached to components thereof in accordance with an aspect of the present disclosure;
FIG. 2 is a perspective view of an anvil assembly of the end effector of the surgical stapling apparatus shown in FIG. 1, showing how a buttress in accordance with an aspect of the present disclosure may be attached thereto;
FIG. 3 is a perspective view of a staple cartridge assembly of the end effector of the surgical stapling apparatus shown in FIG. 1, with some parts thereof separated, showing how a buttress in accordance with an aspect of the present disclosure may be attached thereto;
FIG. 4 is a perspective view of the end effector of FIG. 1 engaged with a tissue to be stapled; and
FIG. 5 is a perspective view of a stapled section of tissue after firing of the surgical stapling apparatus of FIG. 1.

### DETAILED DESCRIPTION

Various exemplary aspects of the present disclosure are discussed herein below in terms of buttresses for use with tissue fixation devices, in aspects surgical staples. While the below disclosure discusses in detail the use of these buttresses with staples, it will be appreciated that surgical stapling apparatuses of the present disclosure include a range of buttressing materials and film-based materials that may be used to mechanically support tissues, reinforce tissues along staple or suture lines, and decrease the incidence of fluid leakage and/or bleeding of tissues. For example, other suitable materials which may be used with the surgical stapling apparatus of the present disclosure include hernia patches and/or tissue scaffolds.

The buttress used with a surgical stapling apparatus of the present disclosure is in the form of a generally rectangular body having a distal end and a proximal end, with opposing lateral sides that run along the length of the elongate rectangular body portion from the distal end to the proximal end.

In aspects, a buttress of the present disclosure may possess a biocompatible contrast agent. Biocompatible contrast agents added to the buttress of the present disclosure are suitable for further identification of cancer cells at or near the site where the buttress of the present disclosure is placed. Thus, the present disclosure describes buttresses, and methods and mechanisms for using the same, for assisting in the removal of cancerous tissue from a patient.

The material used to form the buttress may be impregnated with the biocompatible contrast agent, the biocompatible contrast agent may be applied to the buttress as a coating, or combinations of these applications of biocompatible contrast agent(s) may be used. In other aspects, the biocompatible contrast agent may be a component of fibers used to form the buttress, incorporated into the fibers during the buttress manufacturing process. In some aspects, as set forth in greater detail below, additional layers and/or coatings may be applied to buttresses of the present disclosure.

The biocompatible contrast agent elutes into the tissue once the buttress and staple line is deployed. The elution of the biocompatible contrast agent into the tissue adjacent the resected tissue is three-dimensional and, under a specific light, allows for the visualization of abnormal cells, e.g., cancerous cells. In aspects where the biocompatible contrast agent is a component of fibers used to form the buttress, elution of the biocompatible contrast agent from the buttress will be slower and over a longer period of time, which may be helpful in checking for metastases during follow-up care, i.e., permitting visualization of cancerous cells in the weeks following surgery. This extended elution of the biocompatible contrast agent may thus be beneficial in assisting in radiation treatments, targeting those areas of tissue possessing cancerous cells.

It should be understood that a variety of surgical stapling apparatuses may be utilized with a buttress of the present disclosure. In aspects, linear staplers may be utilized such as, for example, those including EndoGIA^{™} Reinforced Reload with Tri-Staple Technology^{™} and other staplers with Tri-Staple^{™} technology, available through Medtronic, (North Haven, CT), as well as other anastomosis staplers, such as, for example, EEA^{™}, CEEA^{™}, GIA^{™}, EndoGIA^{™}, and TA^{™}, also available through Medtronic. It should also be appreciated that the principles of the present disclosure are equally applicable to surgical staplers having alternate configurations, such as, for example, end-to-end anastomosis staplers having a circular cartridge and anvil (see, e.g., commonly owned U.S. Patent No. 5,915,616, entitled "Surgical Fastener Applying Apparatus," the entire disclosure of which is incorporated by reference herein); laparoscopic staplers (see, e.g., commonly owned U.S. Pat. Nos. 6,330,965 and 6,241,139, each entitled "Surgical Stapling Apparatus," the entire disclosures of each of which are incorporated by reference herein); and transverse anastomosis staplers (see, e.g., commonly owned U.S. Patent Nos. 5,964,394 and 7,334,717, each entitled "Surgical Fastener Applying Apparatus", the entire disclosures of each of which are incorporated by reference herein).

The presently disclosed buttress and surgical stapling apparatus will now be described in detail with reference to the figures wherein like reference numerals identify similar or identical elements. In the following discussion, the terms "proximal" and "trailing" may be employed interchangeably, and should be understood as referring to the portion of a structure that is closer to a clinician during proper use. The terms "distal" and "leading" may also be employed interchangeably, and should be understood as referring to the portion of a structure that is further from the clinician during proper use. As used herein, the term "patient" should be understood as referring to a human subject or other animal, and the term "clinician" should be understood as referring to a doctor, nurse, or other care provider and may include support personnel.

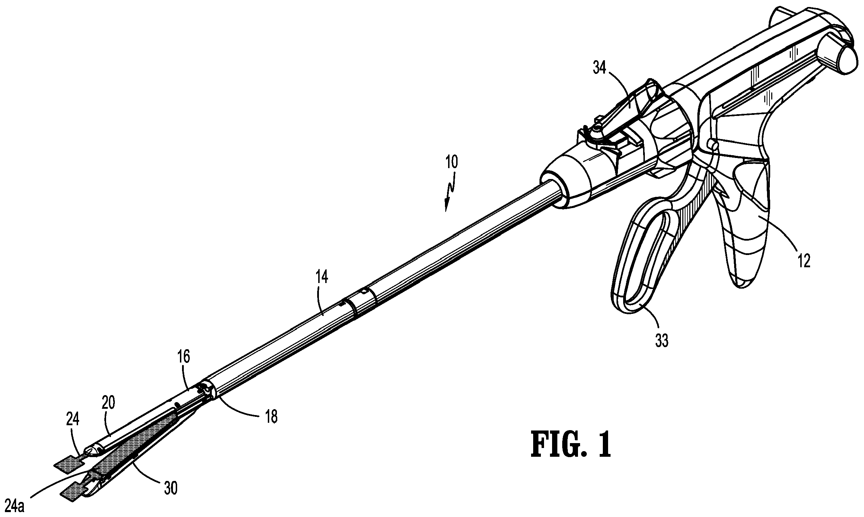

FIGS. 1-3 depict an exemplary surgical stapling device or surgical stapler 10 for use in stapling tissue. As depicted in FIG. 1, the surgical stapling device 10 generally includes a handle 12 and an adapter assembly including an elongate tubular member 14 extending distally from the handle 12. An end effector 16 is mounted on a distal end 18 of the elongate tubular member 14. The end effector 16 may be permanently affixed to the elongate tubular member 14 or may be detachable and thus replaceable with a new end effector 16.

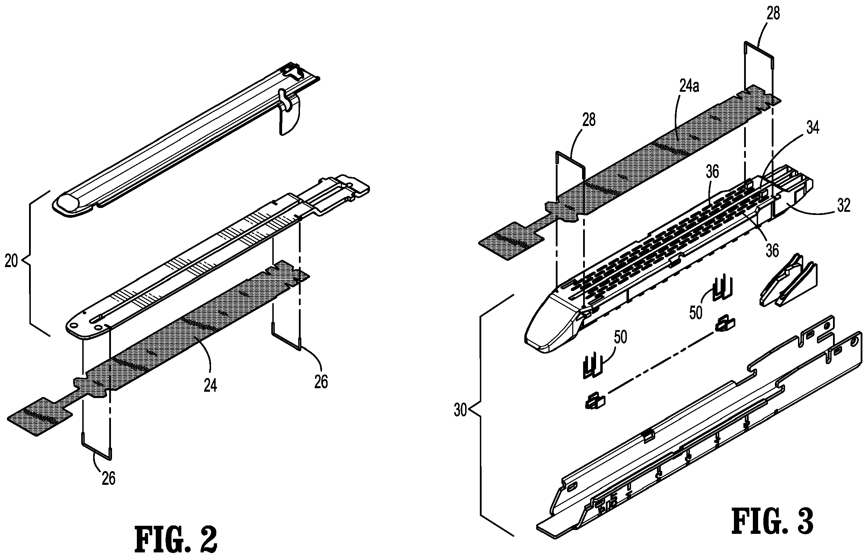

The end effector 16 includes an anvil jaw member 20 (FIG. 2) and a staple cartridge jaw member 30 configured to receive a staple cartridge 32 (FIG. 3). The anvil jaw member 20 is movably mounted on the distal end 18 of the end effector 16 and is movable between an open position spaced apart from the staple cartridge jaw member 30 to a closed position substantially adjacent the staple cartridge jaw member 30.

The staple cartridge jaw member 30 possesses a staple cartridge 32 therein that defines a central knife slot 34 and rows of staple pockets 36 on each side of the central knife slot 34 (FIG 3). It is envisioned that two or more rows of staple pockets 36 can be provided on each side of the central knife slot 34. It is also envisioned that the staple pockets 36 need not be aligned in rows but rather a variety of different arrays of staple pockets 36 can be defined on each side of the central knife slot 34 (not shown).

The surgical stapling apparatus 10 further includes a trigger 33, as seen in FIG. 1, movably mounted on the handle 12. Actuation of the trigger 33 initially operates to move the anvil jaw member 20 from the open to the closed position relative to the staple cartridge jaw member 30 and subsequently actuates the surgical stapling apparatus 10 to apply lines of staples to tissue. In order to properly orient the end effector 16 relative to the tissue to be stapled, the surgical stapling apparatus 10 is additionally provided with a rotation knob 34 mounted on the handle 12. Rotation of the rotation knob 34 relative to the handle 12 rotates the elongate tubular member 14 and the end effector 16 relative to the handle 12 so as to properly orient the end effector 16 relative to the tissue to be stapled.

Referring to FIGS. 2-3, the anvil jaw member 20 and the staple cartridge jaw member 30 may be provided with buttresses 24, 24a. The buttresses 24, 24a are provided to reinforce and seal staple lines applied to tissue by the surgical stapling apparatus 10. The buttresses 24, 24a may be configured in any shape, size, or dimension suitable to fit any surgical stapling, fastening, or firing apparatus.

While FIGS. 2-3 depict buttresses 24, 24a applied to the anvil jaw member 20 and the staple cartridge jaw member 30, respectively, it is to be appreciated that, in some aspects, either the anvil jaw member 20 has the buttress 24 or the staple cartridge jaw member 30 has the buttress 24a, but not both (not shown).

As illustrated in FIG. 2, the buttress 24 may be attached to the anvil jaw member 20 with sutures 26. Similarly, as illustrated in FIG. 3, the buttress 24a may be attached to the staple cartridge jaw member 30 with sutures 28.

As illustrated in FIG. 1, the anvil jaw member 20 has been loaded with a buttress 24, and the staple cartridge jaw member 30 has been loaded with a buttress 24a.

Buttresses in accordance with the present disclosure may be fabricated from a biocompatible substrate material. Such substrates may be formed of bioabsorbable, non-absorbable, natural, and/or synthetic materials.

In aspects, the buttress may be biodegradable, so that the buttress does not have to be retrieved from the body. The term "biodegradable" as used herein is defined to include both bioabsorbable and bioresorbable materials. By biodegradable, it is meant that the buttress decomposes or loses structural integrity under body conditions (e.g., enzymatic degradation or hydrolysis), or is broken down (physically or chemically) under physiologic conditions in the body such that the degradation products are excretable or absorbable by the body.

Non-limiting examples of materials which may be used in forming a buttress of the present disclosure include, but are not limited to, poly(lactic acid), poly(glycolic acid), poly(trimethylene carbonate), poly(dioxanone), poly(hydroxybutyrate), poly(phosphazine), polyethylene terephthalate, polyethylene glycols, polyethylene oxides, polyacrylamides, polyhydroxyethylmethylacrylate, polyvinylpyrrolidone, polyvinyl alcohols, poly acrylic acid, poly acetate, polycaprolactone, polypropylene, aliphatic polyesters, glycerols, poly(amino acids), copoly(ether-esters), polyalkylene oxalates, polyamides, poly(iminocarbonates), polyalkylene oxalates, polyoxaesters, polyorthoesters, polyphosphazenes, and copolymers, block copolymers, homopolymers, blends and combinations thereof.

In aspects, natural biological polymers may be used in forming a buttress of the present disclosure. Suitable natural biological polymers include, but are not limited to, collagen, gelatin, fibrin, fibrinogen, elastin, keratin, albumin, cellulose, oxidized cellulose, hydroxyethyl cellulose, hydroxypropyl cellulose, carboxyethyl cellulose, carboxymethyl cellulose, chitin, chitosan, and combinations thereof. In addition, natural biological polymers may be combined with any of the other polymeric materials described herein to produce a buttress of the present disclosure.

The buttress may also be formed of materials that are porous or non-porous. It should of course be understood that any combination of porous, non-porous, natural, synthetic, bioabsorbable, and/or non-bioabsorbable materials may be used to form a buttress of the present disclosure.

In some aspects, a buttress of the present disclosure may be formed of porous material(s). Any porous portion of a buttress of the present disclosure may have openings or pores over at least a part of a surface thereof. Suitable porous materials include, but are not limited to, fibrous structures (e.g., knitted structures, woven structures, non-woven structures, etc.) and/or foams (e.g., open, or closed cell foams).

In aspects, the pores may be in sufficient number and size so as to interconnect across the entire thickness of the buttress. Woven fabrics, knitted fabrics, non-woven fabrics, and open cell foams are illustrative examples of structures in which the pores can be in sufficient number and size so as to interconnect across the entire thickness of the buttress.

In other aspects, the pores may not interconnect across the entire thickness of the buttress. Closed cell foams or fused non-woven materials are illustrative examples of structures in which the pores may not interconnect across the entire thickness of the buttress. In some aspects, pores may be located on a portion of the buttress, with other portions of the buttress having a non-porous texture. Those skilled in the art may envision a variety of pore distribution patterns and configurations for a porous buttress of the present disclosure.

Where the buttress of the present disclosure is porous and includes fibrous materials, the buttress may be formed using any suitable method including, but not limited to, knitting, weaving, non-woven techniques (including melt blowing), wet-spinning, electro-spinning, extrusion, co-extrusion, and the like. In aspects, the buttress possesses a three-dimensional structure, such as the textiles described in U.S. Patent Nos. 7,021,086 and 6,443,964, the entire disclosures of each of which are incorporated by reference herein.

As depicted in FIG. 1-5, the buttresses 24, 24a have a biocompatible contrast agent. The biocompatible contrast agent may be on a surface of the buttresses 24,24a, within pores of a porous substrate used to form the buttresses 24, 24a, combinations thereof, and the like.

In aspects, suitable biocompatible contrast agents include artificial oxygen carriers. Artificial oxygen carriers nourish cells in need of oxygen. As cancer cells are hypoxic, they will possess greater amounts of the biocompatible contrast agent when compared to healthy tissue.

In aspects, the biocompatible contrast agent included in the buttress will elute into the tissue to which the buttress has been applied during a resection procedure. If, after resection, the remaining tissue in the patient's body has cancer cells, the biocompatible contrast agent will contact the cancer cells and a visual indicator of the presence of the cancer cells will result. For example, upon exposure of the remaining tissue in the patient's body to light of the appropriate wavelength, the cancer cells within the tissue within the patient's body will be dark spots as a "non-consumer" of oxygen. This permits the clinician to visualize additional tissue for resection to ensure all cancerous tissue will be removed from the patient.

Elution of the biocompatible contrast agents from the buttress can be observed while the clinician is waiting for a pathologist report on the resected tissue. This permits confirmation of the presence of cancer cells within the tissue remaining in a patient's boy intraoperatively, in aspects from about 20 minutes to about 30 minutes after resection of the tissue.

Artificial oxygen carriers which may be utilized as biocompatible contrast agents include hemoglobin-based oxygen carriers (HBOCs), perfluorocarbon-based oxygen carriers (PFCs), combinations thereof, and the like. The hemoglobin molecule in hemoglobin based artificial O₂ carriers may need to be stabilized to prevent dissociation of the α2β2-hemoglobin tetramer into ab-dimers, in order to prolong intravascular retention and to eliminate nephrotoxicity. Other modifications serve to decrease O₂ affinity in order to improve O₂ off-loading to tissues. In addition, polyethylene glycol may be surface conjugated to the hemoglobin molecule of hemoglobin based artificial O₂ carriers to increase molecular size. Finally, certain products are polymerized to increase the hemoglobin concentration at physiologic colloid oncotic pressure. Perfluorocarbons are carbon-fluorine compounds characterized by a high gas dissolving capacity for O₂ and CO₂ and chemical and biologic inertness.

Commercially available examples of artificial oxygen carriers include diaspirin cross-linked hemoglobin (HemAssist), human recombinant hemoglobin (rHb1.1 and rHb2.0), polymerized bovine hemoglobin-based O₂ carrier (HBOC-201), human polymerized hemoglobin (PolyHeme^{®}), hemoglobin raffimer (Hemolink^{™}), maleimide-activated polyethylene glycol-modified hemoglobin (MP4), and perfluorocarbon emulsions such as those available under the trade names Oxygent^{™}, and OxyFluor^{™}.

The biocompatible contrast agent, in aspects an artificial oxygen carrier, may be detected in any remaining cancer cells withing the patient's tissue with light of an appropriate wavelength. In aspects, the artificial oxygen carrier utilized as the biocompatible contrast agent may possess a fluorophore or similar element/compound which enhances visualization of the artificial oxygen carrier under light of a specific wavelength.

In aspects, the biocompatible contrast agent applied to the substrate material in forming a buttress of the present disclosure may also include excipients to enhance both the ability of the biocompatible contrast agent to adhere to the buttress, as well as to modify the elution of the biocompatible contrast agent from the buttress.

In aspects, suitable excipients which may be combined with a biocompatible contrast agent in forming the buttress include surfactants such as, but not limited to, cyclodextrins such as 2-hydroxypropyl-beta-cyclodextrin and methyl-B-cyclodextrin, sodium dodecyl sulfate, octyl glucoside, and sorbitan fatty acid esters such as sorbitan monooleate, sorbitan monolaurate and polyethoxylated fatty acid esters of sorbitan, sometimes referred to herein as polysorbates, including those sold under the name TWEEN^{™}. Examples of such polysorbates include polysorbate 80 (TWEEN^{™} 80), polysorbate 20 (TWEEN^{™} 20), polysorbate 60 (TWEEN^{™} 60), polysorbate 65 (TWEEN^{™} 65), polysorbate 85 (TWEEN^{™} 85), combinations thereof, and the like. In aspects, low molecular weight poly(ethylene glycol)s may be added as an excipient, either alone or in any combination with any of the other above excipients.

In other aspects, suitable excipients may include salts such as sodium chloride and/or other materials such as urea, oleic acid, citric acid, and ascorbic acid. In yet other aspects, the excipient may be a stabilizer such as butylated hydroxytoluene (BHT) or butylated hydroxyanisole (BHA).

Still other suitable excipients include polyhydric alcohols such as D-sorbitol, mannitol, combinations thereof, and the like.

In some aspects, excipients which are hydrotropes may be included with the biocompatible contrast agent(s). These materials attract water, which may enhance the release of the biocompatible contrast agent from the buttress.

In aspects, the biocompatible contrast agent(s), carrier component(s) and/or excipient(s) may be in a solution for application to a buttress of the present disclosure. Any suitable solvent that can be removed by drying may be used to form such a solution. Suitable solvents for forming such a solution include any pharmaceutically acceptable solvents including, but not limited to, saline, water, alcohol, acetone, dimethyl sulfoxide, ethyl acetate, N-methylpyrrolidone, combinations thereof, and the like. Methods for forming such solutions are within the purview of those skilled in the art and include, but are not limited to, mixing, blending, sonication, heating, combinations thereof, and the like.

In aspects, the biocompatible contrast agent(s), any carrier component(s), and/or any excipient(s) may be applied to the buttress by spraying, dipping, brushing, a needle deposition process, combinations thereof, and the like. As noted above, in aspects the biocompatible contrast agent is in a solution, which is then applied to a buttress.

In aspects, the biocompatible contrast agent(s), any carrier component(s), and/or any excipient(s) may be applied to the medical device of the present disclosure prior to affixing the medical device to some other medical apparatus. For example, in the case of a buttress, the biocompatible contrast agent may be applied to the buttress prior to its attachment to a surgical stapler.

In accordance with the present disclosure, the biocompatible contrast agent(s), solvent(s), any carrier component(s), and/or any excipient(s), are applied so that an adequate amount of biocompatible contrast agent(s) is deposited and stays robustly attached to the buttress.

After application, the solvent from the coating solution may be driven off by methods within the purview of those skilled in the art. For example, solvent evaporation may be facilitated by heat, gas flow, time, reduced pressure, combinations thereof, and the like, to increase the accuracy of drug deposition on the buttress. Moreover, this assisted evaporation of solvent may be applied to the whole surface of the buttress, or partially to only a portion of the surface of the buttress.

Driving off the solvent leaves the biocompatible contrast agent and any carrier component and/or excipient behind on the buttress or within the pores of a porous buttress.

In some aspects, while not depicted, the buttresses 24, 24a may have a gradient of biocompatible contrast agent thereon/therein, with a greater concentration of the biocompatible contrast agent at the opposing lateral sides that run along the length of the elongate rectangular body portion of the buttress, with a lower concentration of the biocompatible contrast agent at the central portion of the buttresses 24, 24a.

After formation, buttress of the present disclosure may possess the biocompatible contrast agent amounts from about 0.1 % to about 50 % by weight of the coated buttress, in aspects from about 1 % to about 10 % by weight of the coated buttress. While excipients are not required, where present, non-polymeric excipients may be present in an amount from about 0.01 % to about 80% by weight of the coated buttress, in aspects from about 1 % to about 11 % by weight of the coated buttress. In other aspects, where present, polymeric excipients may be present in an amount from about 0.014% to about 14% by weight of the coated buttress, in aspects from about 5% to about 15% by weight of the coated buttress.

The porosity of the fabric used to form the substrate may allow for the infiltration of biological fluids and/or cellular components which, in turn, may accelerate the release kinetics of the biocompatible contrast agent from the buttress of the present disclosure, thus increasing the rate of release of biocompatible contrast agent(s) from the buttress into the surrounding tissue and fluids.

Substrates used to form buttresses of the present disclosure may have a thickness from about 0.05 mm to about 0.5 mm, in aspects from about 0.1 mm to about 0.2mm.

Where the substrate used to form the buttress is porous, the buttress of the present disclosure may have a pore volume from about 65% to about 85%, in aspects from about 70% to about 80%.

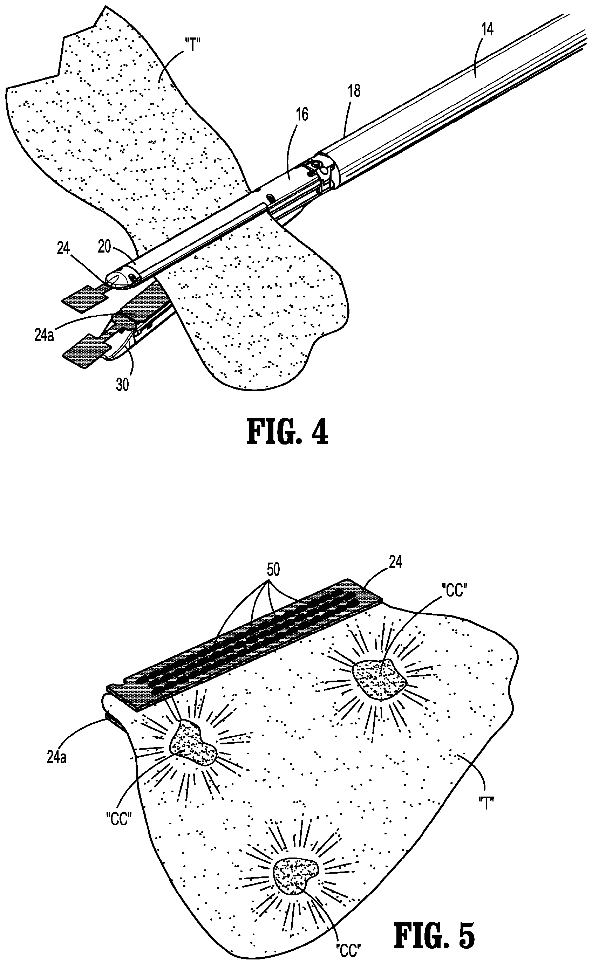

In use, as generally depicted in FIGS. 4-5, the buttresses 24, 24a described herein may be used in sealing a wound by approximating the edges of wound tissue between the staple cartridge jaw member 30 and the anvil jaw member 20 of the surgical stapling apparatus 10. Firing of the surgical stapling apparatus 10 forces at least one staple 50 to pass through the openings on the staple cartridge jaw member 30, the buttress 24a on the staple cartridge jaw member 30, the tissue "T", the buttress 24 on the anvil jaw member 20, and the openings on the anvil (not shown) to secure the buttresses 24, 24a to the tissue "T" so that the tissue is sandwiched between the two, thereby securing the adjoining tissue and to seal the tissue.

The resulting tissue "T", divided and stapled closed with staples 50, is illustrated in FIG. 5. Specifically, the buttress 24a associated with the staple cartridge jaw member 30 and the buttress 24 associated with the anvil jaw member 20 is secured against tissue "T" by the staples 50, thereby sealing and reinforcing the staple line created by staples 50. After resection, the biocompatible contrast agent elutes into the tissue "T" remaining in the patient. Cancer cells "CC" in the tissue "T" will absorb the biocompatible contrast agent, and thus be of a darker hue, thereby allowing the clinician to visually observe the presence of such cancer cells "CC", while awaiting the biopsy results for such tissue that has already been removed as part of the resection.

While the above description is directed to rectangular buttresses, it is to be appreciated that any suitable configuration for a buttress may be utilized in accordance with the present disclosure. For example, buttresses having an elongate rectangular body with head and tail portions at the ends of the buttress may be utilized. Any other suitable shape may be utilized. For example, additional suitable buttresses include those disclosed in U.S. Patent Application Publication No. 2017/0296188, and U.S. Patent Nos. 8,157,151, 8,561,873 and 9,693,772, the entire disclosures of each of which are incorporated by reference herein.

In aspects, the buttress may also include therapeutic agents. Suitable therapeutic agents which may be added to a buttress include, but are not limited to, drugs, amino acids, peptides, polypeptides, proteins, polysaccharides, muteins, immunoglobulins, antibodies, cytokines (e.g., lymphokines, monokines, chemokines), blood clotting factors, hemopoietic factors, interleukins (1 through 18), interferons (β-IFN, α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, fibrin, thrombin, fibrinogen, synthetic thrombin, synthetic fibrin, synthetic fibrinogen, gonadotropins (e.g., FSH, LH, CG, etc.), hormones and hormone analogs (e.g., growth hormone, luteinizing hormone releasing factor), vaccines (e.g., tumoral, bacterial and viral antigens), somatostatin, antigens, blood coagulation factors, growth factors (e.g., nerve growth factor, insulin-like growth factor), bone morphogenic proteins, TGF-B, protein inhibitors, protein antagonists, protein agonists, nucleic acids, such as antisense molecules, DNA, RNA, RNAi, oligonucleotides, polynucleotides, cells, viruses, anti-inflammatory agents, anti-bacterial agents, antimicrobial agents, and ribozymes.

The medical devices of the disclosure and its associated methods of use have several advantages including, for example:
- Addition of a fast-eluting biocompatible contrast agent to the buttress permits diagnosis of tissue adjacent the staple line;
- The biocompatible contrast agent may be used intraoperatively in combination with a light for detection of cancer cells beyond the resection site;
- Intraoperative detection complementing pathology analysis to guide surgical decision and post-operative therapy;
- Fast-eluting biocompatible contrast agent that will differentially diffuse and contrast healthy tissue vs. cancerous tissue; and
- Complete removal of the cancer cells means less aggressive postoperative radiation and chemotherapy treatments, which will allow for quicker patient recovery.

It will be understood that various modifications may be made to the aspects disclosed herein. Therefore, the above description should not be construed as limiting, but merely as an exemplification of preferred aspects. Those skilled in the art will envision other modifications within the scope and spirit of the present disclosure. Such modifications and variations are intended to come within the scope of the following claims.

The invention may be described by reference to the following numbered paragraphs:-
1. A surgical stapling device, comprising:
   an end effector including an anvil jaw member and a staple cartridge jaw member pivotally coupled to one another, the anvil jaw member and the staple cartridge jaw member being relatively movable such that the end effector is movable between an open position and a clamped position; and
   a first buttress attached to the anvil jaw member, the first buttress including at least one biocompatible contrast agent.
2. The surgical stapling device of paragraph 1, further comprising a second buttress attached to the cartridge jaw member, the second buttress including at least one biocompatible contrast agent.
3. The surgical stapling device of paragraph 1, wherein the at least one biocompatible contrast agent is an artificial oxygen carrier selected from hemoglobin-based oxygen carriers, perfluorocarbon-based oxygen carriers, or combinations thereof.
4. The surgical stapling device of paragraph 1, wherein the biocompatible contrast agent is combined with an excipient including a surfactant, a salt, an acid, a stabilizer, a polyhydric alcohol, a hydrotrope, a low molecular weight poly(ethylene glycol) or any combination thereof.
5. The surgical stapling device of paragraph 4, wherein the excipient includes a surfactant selected from cyclodextrin, sodium dodecyl sulfate, octyl glucoside, a sorbitan fatty acid ester, or combinations thereof.
6. The surgical stapling device of paragraph 4, wherein the excipient includes a salt comprising sodium chloride.
7. The surgical stapling device of paragraph 4, wherein the excipient includes an acid selected from oleic acid, citric acid, ascorbic acid, or combinations thereof.
8. The surgical stapling device of paragraph 4, wherein the excipient includes a stabilizer selected from butylated hydroxytoluene, or butylated hydroxyanisole.
9. The surgical stapling device of paragraph 4, wherein the excipient includes a polyhydric alcohol selected from D-sorbitol, mannitol, or combinations thereof.
10. The surgical stapling device of paragraph 1, wherein the first buttress is attached to the anvil jaw member by at least one suture.
11. The surgical stapling device of paragraph 2, wherein the second buttress is attached to the staple cartridge jaw member by at least one suture.
12. The surgical stapling device of paragraph 1, wherein the first buttress further comprises a therapeutic agent selected from amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs, vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, anti-inflammatory agents, anti-bacterial agents, antimicrobial agents, and ribozymes.
13. A method for treating tissue comprising stapling tissue with the surgical stapling device of paragraph 1.
14. A surgical stapling device, comprising:
   an end effector including an anvil jaw member and a staple cartridge jaw member pivotally coupled to one another, the anvil jaw member and the staple cartridge jaw member being relatively movable such that the end effector is movable between an open position and a clamped position;
   a first buttress attached to the anvil jaw member, the first buttress having at least one biocompatible contrast agent; and
   a second buttress attached to the cartridge jaw member, the second buttress having at least one biocompatible contrast agent.
15. The surgical stapling device of paragraph 14, wherein the at least one biocompatible contrast agent includes an artificial oxygen carrier selected from hemoglobin-based oxygen carriers, perfluorocarbon-based oxygen carriers, or combinations thereof.
16. The surgical stapling device of paragraph 14, wherein the biocompatible contrast agent is combined with an excipient including a surfactant, a salt, an acid, a stabilizer, a polyhydric alcohol, a hydrotrope, a low molecular weight poly(ethylene glycol) or any combination thereof.
17. The surgical stapling device of paragraph 14, wherein the first buttress is attached to the anvil jaw member by at least one suture.
18. The surgical stapling device of paragraph 14, wherein the second buttress is attached to the staple cartridge jaw member by at least one suture.
19. The surgical stapling device of paragraph 14, wherein the first buttress, the second buttress, or both, further comprise a therapeutic agent selected from amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs, vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, anti-inflammatory agents, anti-bacterial agents, antimicrobial agents, or ribozymes.
20. A method for treating tissue comprising stapling tissue with the surgical stapling device of paragraph 14.

## Claims

1. A surgical stapling device, comprising:
an end effector including an anvil jaw member and a staple cartridge jaw member pivotally coupled to one another, the anvil jaw member and the staple cartridge jaw member being relatively movable such that the end effector is movable between an open position and a clamped position; and
a first buttress attached to the anvil jaw member, the first buttress including at least one biocompatible contrast agent.

2. The surgical stapling device of claim 1, further comprising a second buttress attached to the cartridge jaw member, the second buttress including at least one biocompatible contrast agent.

3. The surgical stapling device of claim 1 or claim 2, wherein the at least one biocompatible contrast agent is an artificial oxygen carrier selected from hemoglobin-based oxygen carriers, perfluorocarbon-based oxygen carriers, or combinations thereof.

4. The surgical stapling device of any preceding claim, wherein the biocompatible contrast agent is combined with an excipient including a surfactant, a salt, an acid, a stabilizer, a polyhydric alcohol, a hydrotrope, a low molecular weight poly(ethylene glycol) or any combination thereof; preferably wherein the excipient includes a surfactant selected from cyclodextrin, sodium dodecyl sulfate, octyl glucoside, a sorbitan fatty acid ester, or combinations thereof.

5. The surgical stapling device of claim 4, wherein the excipient includes a salt comprising sodium chloride; and/or wherein the excipient includes an acid selected from oleic acid, citric acid, ascorbic acid, or combinations thereof; and/or wherein the excipient includes a stabilizer selected from butylated hydroxytoluene, or butylated hydroxyanisole; and/or wherein the excipient includes a polyhydric alcohol selected from D-sorbitol, mannitol, or combinations thereof.

6. The surgical stapling device of any preceding claim, wherein the first buttress is attached to the anvil jaw member by at least one suture.

7. The surgical stapling device of claim 2, wherein the second buttress is attached to the staple cartridge jaw member by at least one suture.

8. The surgical stapling device of any preceding claim, wherein the first buttress further comprises a therapeutic agent selected from amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs, vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, anti-inflammatory agents, anti-bacterial agents, antimicrobial agents, and ribozymes.

9. A surgical stapling device, comprising:
an end effector including an anvil jaw member and a staple cartridge jaw member pivotally coupled to one another, the anvil jaw member and the staple cartridge jaw member being relatively movable such that the end effector is movable between an open position and a clamped position;
a first buttress attached to the anvil jaw member, the first buttress having at least one biocompatible contrast agent; and
a second buttress attached to the cartridge jaw member, the second buttress having at least one biocompatible contrast agent.

10. The surgical stapling device of claim 9, wherein the at least one biocompatible contrast agent includes an artificial oxygen carrier selected from hemoglobin-based oxygen carriers, perfluorocarbon-based oxygen carriers, or combinations thereof.

11. The surgical stapling device of claim 9 or claim 10, wherein the biocompatible contrast agent is combined with an excipient including a surfactant, a salt, an acid, a stabilizer, a polyhydric alcohol, a hydrotrope, a low molecular weight poly(ethylene glycol) or any combination thereof.

12. The surgical stapling device of any of claims 9 to 11, wherein the first buttress is attached to the anvil jaw member by at least one suture.

13. The surgical stapling device of any of claims 9 to 12, wherein the second buttress is attached to the staple cartridge jaw member by at least one suture.

14. The surgical stapling device of any of claims 9 to 13, wherein the first buttress, the second buttress, or both, further comprise a therapeutic agent selected from amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs, vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, anti-inflammatory agents, anti-bacterial agents, antimicrobial agents, or ribozymes.
